# EUROPÄISCHE PATENTSCHRIFT

(11) **EP 0 415 075 B1**
(45) Veröffentlichungstag und Bekanntmachung des Hinweises auf die Patenterteilung: **12.10.1994**
(21) Anmeldenummer: 90114093.9
(22) Anmeldetag: 23.07.1990
(51) Int. Cl.: A61B 6/14

(54) **Zahnärztliche Röntgendiagnostikeinrichtung**
Dental X-ray diagnostic apparatus
Appareil radiographique destiné au diagnostic dentaire

(30) Priorität: 03.08.1989 DE 8909398 U
(43) Veröffentlichungstag der Anmeldung: 06.03.1991
(73) Patentinhaber: SIEMENS AKTIENGESELLSCHAFT, 80333 München (DE)
(72) Erfinder: Pfeiffer, Joachim, Dr., D-6140 Bensheim (DE); Schulze-Ganzlin, Ulrich, Dipl.-Ing., D-6143 Lorsch (DE); Schwotzer, Axel, Dipl.-Ing., D-6087 Büttelborn 1 (DE)

(56) Entgegenhaltungen:
- EP-A- 0 279 293
- EP-A- 0 372 122
- DE-A- 2 443 681
- FR-A- 2 309 196
- FR-A- 2 596 603

## Beschreibung

Die Erfindung bezieht sich auf eine zahnärztliche Röntgendiagnostikeinrichtung, enthaltend einen Röntgenstrahler zur Durchstrahlung eines Objektes (Kiefer, Zahn) vom Schädel eines Patienten, einen diametral zum Röntgenstrahler und in Strahlungsrichtung nach dem zu durchstrahlenden Objekt angeordneten Bilddetektor, eine mit dem Bilddetektor verbundene Steuerelektronik mit einem den Bilddetektor ansteuernden Steuerteil und einem Bildverarbeitungsteil, der die von dem Bilddetektor gewonnenen Signale aufarbeitet und einer Anzeigevorrichtung zuführt, sowie eine Bedienvorrichtung, mit der der Röntgenstrahler ein- und ausschaltbar ist und ggfs. weitere Steuervorgänge schaltbar sind.

Eine Röntgendiagnostikeinrichtung dieser Art kann sowohl eine sogenannte Intraoral-Röntgendiagnostikeinrichtung sein, bei der die Röntgenstrahlenquelle außerhalb und der Bilddetektor im Patientenmund angeordnet ist, als auch eine Röntgendiagnostikeinrichtung, bei der die Röntgenstrahlenquelle innerhalb und der Bilddetektor außerhalb des Patientenmundes angeordnet ist.

Nachdem der grundsätzliche Aufbau beider Systeme bekannt ist, wird davon abgesehen, diese im einzelnen näher zu erläutern. Eine Intraoral-Röntgendiagnostikeinrichtung ist beispielsweise in der EP-A-0 129 451 beschrieben.

Davon ausgehend, daß Röntgenstrahler und Bildempfangssystem räumlich getrennte Einheiten sind, ist es notwendig, zur Synchronisation des Aufnahmevorganges den Steuerteil des Röntgenstrahlers mit der Steuerelektronik des Empfangssystems zu verbinden. Dies hat jedoch den Nachteil, daß beide Steuerteile aufeinander abgestimmt sein müssen und daß zwischen den räumlich getrennten Baueinheiten ein zusätzliches Verbindungskabel vorhanden ist, welches die Mobilität der Diagnostikeinrichtung, insbesondere bei Verwendung als Intraoral-Röntgendiagnostikeinrichtung, beeinträchtigt.

Der im Anspruch 1 angegebenen Erfindung liegt die Aufgabe zugrunde, demgegenüber eine Verbesserung zu schaffen, insbesondere mit dem Ziel, auf das vorgenannte Verbindungskabel zwischen Steuerteil des Röntgenstrahlers und der Empfangseinheit verzichten zu können.

Dadurch, daß gemäß der Erfindung ein strahlenempfindlicher Sensor im Empfangsbereich der Strahlung des Bilddetektors angeordnet ist, kann dieser kontinuierlich betrieben werden.

Nach dem Stand der Technik ist dies nicht möglich, da die dort verwendeten Bilddetektoren nach dem Frame-Transfer-Mode arbeiten, bei dem zyklisch in Abständen von maximal einer Sekunde die gesamte Bildinformation aus dem Bilddetektor ausgelesen sein muß. Dieser Vorgang dauert einen Bruchteil einer Sekunde. Während dieser Zeit ist jedoch eine aktive Bilderfassung nicht möglich. Eine unterbrechungsfreie Bilderfassung ist somit beim Stand der Technik nicht gewährleistet. Der gemäß der Erfindung vom Bilddetektor unabhängige röntgenstrahlenempfindliche Sensor erfaßt nach Einschalten des Röntgenstrahlers den Beginn der Strahlung, wodurch der Bilddetektor zur Bildintegration aktiviert wird, und zwar nur solange tatsächlich Strahlung auftritt. Die Bildintegrationsphase wird auf die tatsächliche Strahlungszeit begrenzt; Ruhestromeinflüsse außerhalb der Strahlungszeit können somit weitgehend unterdrückt werden.

Der röntgenstrahlungsempfindliche Sensor kann in Form eines oder mehrerer Phototransistoren oder einem entsprechenden anderen elektronischen Bauelement realisiert sein, denen eine Szintillatorschicht vorgelagert ist. Der Sensor kann vorteilhafterweise unmittelbar neben oder auch hinter der aktiven Fläche des Bilddetektors angeordnet oder als eigenständiges Element in die Fläche des Bilddetektors integriert sein; denkbar ist es auch, den Sensor in den Silizium- oder Keramikträger des Bilddetektors zu integrieren.

Nachfolgend wird ein Ausführungsbeispiel der Erfindung beschrieben.

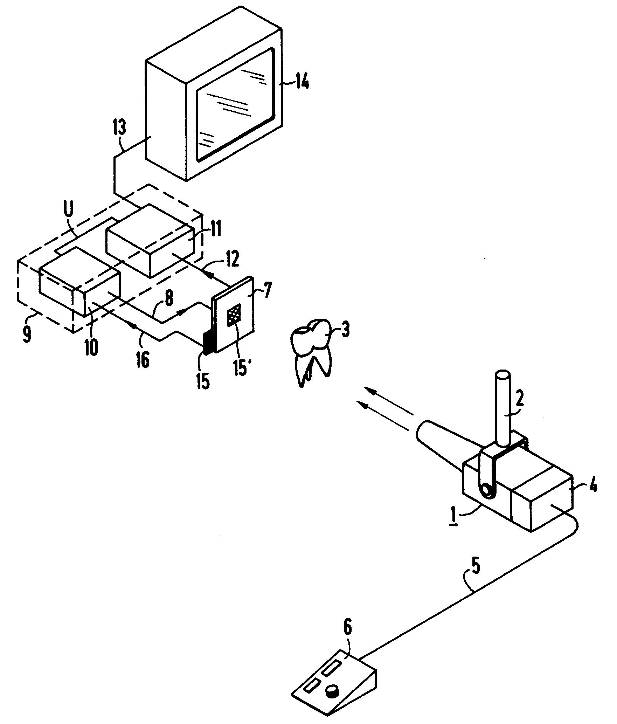

Die Zeichnung zeigt in einer schematisierten Darstellung den Aufbau einer zahnärztlichen Röntgendiagnostikeinrichtung nach der Erfindung.

Ein Röntgenstrahler 1, der in bekannter Weise eine nicht näher bezeichnete Röntgenröhre mit Hochspannungsgenerator enthält, ist mittels einer Halterung 2 in geeigneter Weise so gehaltert, daß er auf das zu durchstrahlende Objekt 3 (Zahn bzw. Kiefer eines Patienten) ausrichtbar ist.

Die Halterung 2 besteht im Falle einer Intraoral-Röntgendiagnostikeinrichtung in bekannter Weise aus einem (Boden- oder Decken-) Stativ, an dem der Röntgenstrahler in Höhe, Neigung und Schwenkrichtung einstellbar gehaltert ist.

Der Röntgenstrahler 1 enthält ferner ein mit 4 bezeichnetes Steuerteil, welches über ein Steuerkabel 5 mit einer Bedieneinheit 6 verbunden ist, mit deren Hilfe bestimmte Aufnahmewerte (Röhrenspannung, Belichtungszeit) einstellbar sind.

Dem Röntgenstrahler diametral gegenüberliegend und in Strahlungsrichtung nach dem Objekt 3 ist ein allgemein mit 7 bezeichneter Bilddetektor angeordnet. Dieser Bilddetektor ist im Falle einer Intraoral-Röntgendiagnostikeinrichtung im Patientenmund, also intraoral, plazierbar.

Der Bilddetektor 7 besteht vorteilhafterweise aus einem CCD-Element, welches über eine Steuerleitung 8 mit einer Steuerelektronik 9 verbunden ist. Die Steuerelektronik 9 enthält einen Steuerteil 10 sowie einen Bildverarbeitungsteil 11. Der Steuerteil 10 enthält unter anderem einen Taktgenerator zur Ansteuerung des CCD-Detektors. Die vom Bilddetektor 7 gewonnenen Signale werden über eine Steuerleitung 12 dem Bildverarbeitungsteil zugeführt, der die Signale aufbereitet, verstärkt und anschließend über eine Leitung 13 einem Monitor 14 zuführt.

Mit 15 ist ein strahlungsempfindlicher Sensor bezeichnet, der unmittelbar benachbart der aktiven Fläche des Bilddetektors 7 angeordnet ist. Der Sensor erfaßt die von Röntgenstrahler 1 ausgehende Strahlung in einem Punkt und gibt über eine Steuerleitung 16 eine Information an den Steuerteil 10, welcher sodann über die Steuerleitung 8 den Bilddetektor 7 bei Vorhandensein von Strahlung aktiviert. Der Sensor kann aus ein oder mehreren Phototransistoren mit vorgelagerter Szintillationsschicht bestehen; es ist aber auch denkbar, andere röntgenstrahlenempfindliche Sensoren einzusetzen. Wenngleich es vorteilhaft ist, den Sensor unmittelbar benachbart der aktiven Fläche des Bilddetektors anzuordnen, so ist es aber auch denkbar, bei strahlungsdurchlässigen Bilddetektoren den Sensor hinter der aktiven Fläche des Bilddetektors anzuordnen. Auch eine Integration des Sensors in den Bilddetektor ist möglich, indem dieser als eigenständiges Element auf dem Silizum- oder Keramikträger des Bilddetektors aufgebracht ist. Eine solche Alternativlösung ist in der Darstellung mit der Pos. 15′ bezeichnet.

## Patentansprüche

1. Zahnärztliche Röntgendiagnostikeinrichtung, enthaltend einen Röntgenstrahler (1), einen diametral dazu und in Strahlungsrichtung nach dem zu durchstrahlenden Objekt (3) angeordneten Bilddetektor (7), eine mit dem Bilddetektor (7) verbundene Steuerelektronik (9) mit einem Steuerteil (10), welches den Bilddetektor (7) ansteuert und einem Bildverarbeitungsteil (11), welcher die vom Bilddetektor (7) gewonnenen Signale aufarbeitet und einer Anzeigevorrichtung (14) zu führt, sowie eine Bedienvorrichtung (6), mit der der Röntgenstrahler (1) ein- und ausschaltbar ist und mit dem ggfs. weitere Steuervorgänge schaltbar sind, **dadurch gekennzeichnet,** daß im Strahlenempfangsbereich des Bilddetektors (7) ein strahlungsempfindlicher Sensor (15, 15′) angeordnet ist, welcher bei Vorhandensein von Strahlung den Steuerteil (10) des Bilddetektors (7) aktiviert.

2. Röntgendiagnostikeinrichtung nach Anspruch 1, **dadurch gekennzeichnet,** daß der Sensor (15) unmittelbar benachbart der aktiven Fläche des Bilddetektors (7) angeordnet ist.

3. Röntgendiagnostikeinrichtung nach Anspruch 1, **dadurch gekennzeichnet,** daß der Sensor (15′) innerhalb der Fläche des Bilddetektors (7) angeordnet ist.

4. Röntgendiagnostikeinrichtung nach Anspruch 3, **dadurch gekennzeichnet,** daß der Sensor (15′) hinter der strahlungsdurchlässig ausgebildeten Fläche des Bilddetektors (7) angeordnet ist.

5. Röntgendiagnostikeinrichtung nach einem der Ansprüche 1 bis 4, **dadurch gekennzeichnet,** daß als Bilddetektor (7) ein CCD-Wandler vorgesehen ist.

6. Röntgendiagnostikeinrichtung nach einem der Ansprüche 1 bis 5, **dadurch gekennzeichnet,** daß Bilddetektor (7) und Sensor (15) integraler Bestandteil eines intraoral eines Patienten angeordneten Empfangssystems sind.

7. Röntgendiagnostikeinrichtung nach einem der Ansprüche 1 bis 6, **dadurch gekennzeichnet,** daß der Sensor (15) aus ein oder mehreren Phototransistoren mit vorgelagerter Szintillationsschicht besteht.

## Claims

1. A dental X-ray diagnostic apparatus, comprising an X-ray source (1), an image detector (7) arranged diametrically in relation to the said source and, in the radiation direction, after the object (3) through which radiation is to pass, an electronic control unit (9) connected to the image detector (7) with a control part (10) which controls the image detector (7) and an image processing part (11) which processes the signals obtained from the image detector (7) and supplies them to a display device (14), and an operating device (6) with which the X-ray source (1) can be switched on and off and, if appropriate, other control procedures can be actuated, characterized in that a radiation-sensitive sensor (15, 15') is arranged in the radiation receiving region of the image detector (7) which in the presence of radiation activates the control part (10) of the image detector (7).

2. An X-ray diagnostic apparatus according to claim 1, characterized in that the sensor (15) is arranged immediately adjacent to the active surface of the image detector (7).

3. An X-ray diagnostic apparatus according to claim 1, characterized in that the sensor (15') is arranged within the surface of the image detector (7).

4. An X-ray diagnostic apparatus according to claim 3, characterized in that the sensor (15') is arranged behind the surface of the image detector (7) which is designed to be permeable to radiation.

5. An X-ray diagnostic apparatus according to one of claims 1 to 4, characterized in that a CCD-converter is provided as the image detector (7).

6. An X-ray diagnostic apparatus according to one of claims 1 to 5, characterized in that the image detector (7) and the sensor (15) are an integral component part of a receiving system arranged intraorally in a patient.

7. An X-ray diagnostic apparatus according to one of claims 1 to 6, characterized in that the sensor (15) is composed of one or more phototransistors with a preceding scintillation layer.

## Revendications

1. Appareil de radiodiagnostic dentaire, contenant un émetteur radiologique (1), un détecteur d'images (7) diamétralement opposé à cet émetteur et, dans la direction de rayonnement vers l'objet à irradier (3), un système électronique de commande (9) relié au détecteur d'images (7) et comportant une partie de commande (10) qui commande le détecteur d'images (7), et une partie de traitement d'images (11), qui traite les signaux délivrés par le détecteur d'images (7) et les envoie à un dispositif d'affichage (14), ainsi qu'un dispositif de commande (6) au moyen duquel l'émetteur radiologique (1) peut être branché et débranché, et à l'aide duquel éventuellement d'autres processus de commande peuvent être commutés, caractérisé par le fait que dans la zone de réception du rayonnement du détecteur d'images (7) est disposé un capteur (15, 15') sensible au rayonnement qui, dans le cas de la présence d'un rayonnement, active la partie de commande (10) du détecteur d'images (7).

2. Appareil de radiodiagnostic suivant la revendication 1, caractérisé par le fait que le capteur (15) est disposé au voisinage direct de la surface active du détecteur d'images (7).

3. Appareil de radiodiagnostic suivant la revendication 1, caractérisé par le fait que le capteur (15') est disposé dans la surface du détecteur d'images (7).

4. Appareil de radiodiagnostic suivant la revendication 3, caractérisé par le fait que le capteur (15') est disposé en arrière de la surface, agencée de manière à être transparente pour le rayonnement, du détecteur d'images (7).

5. Appareil de radiodiagnostic suivant l'une des revendications 1 à 4, caractérisé par le fait qu'un transducteur CCD est prévu en tant que détecteur d'images (7).

6. Appareil de radiodiagnostic suivant l'une des revendications 1 à 5, caractérisé par le fait que le détecteur d'images (7) et le capteur (15) font partie intégrante d'un système de réception placé dans une position intra-orale chez un patient.

7. Appareil de radiodiagnostic suivant l'une des revendications 1 à 6, caractérisé par le fait que le capteur (1 à 15) est constitué par plusieurs phototransistors, en amont desquels est branchée une couche de scintillation.
